# FASCICULE DE BREVET EUROPEEN

(11) **EP 1 036 572 B1**
(45) Date de publication et mention de la délivrance du brevet: **02.11.2005**
(21) Numéro de dépôt: 00400697.9
(22) Date de dépôt: 14.03.2000
(51) Int. Cl.: A61N 1/05

(54) **Sonde de stimulation de l'oreillette gauche implantable dans le réseau veineux coronarien**
In Koronarvenen implantierbare Leitung zur Stimulation eines linken Herzvorhofs
Cardiac lead implantable in the coronary veins for stimulating the left atria

(30) Priorité: 17.03.1999 FR 9903321
(43) Date de publication de la demande: 20.09.2000
(73) Titulaire: ELA MEDICAL (Société anonyme), 92541 Montrouge (FR)
(72) Inventeur: Ollivier, Jean-François, 91190 Villiers le Bade (FR)
(74) Mandataire: Dupuis-Latour, Dominique

(56) Documents cités:
- EP-A- 0 159 753
- WO-A-92/21278
- WO-A-95/26678
- US-A- 4 579 119
- US-A- 5 755 761

## Description

L'invention concerne les sondes de stimulation cardiaque, notamment celles destinées à être implantées dans le réseau coronarien du coeur pour permettre la stimulation de l'oreillette gauche par un "dispositif médical implantable actif" tel que défini par la directive 90/385/CEE du 20 juin 1990 du Conseil des communautés européennes, et plus précisément un dispositif tel qu'un stimulateur cardiaque, défibrillateur et/ou cardioverteur, notamment un stimulateur de type "multisite".

À la différence de l'oreillette droite ou du ventricule droit pour lesquels il suffit d'implanter des sondes endocavitaires via le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du coeur gauche.

C'est pour cette raison que l'on choisit dans ce cas une sonde introduite dans le réseau coronarien, l'accès à l'entrée du sinus coronaire se faisant via l'oreillette droite. Cette sonde est généralement pourvue à son extrémité distale d'une électrode disposée face au ventricule gauche ; en complément ou en variante, elle peut également être pourvue d'une électrode disposée face à l'oreillette gauche.

L'invention a pour objet la réalisation d'une telle sonde, pourvue d'une électrode de stimulation de l'oreillette gauche.

La stimulation de l'oreillette gauche depuis le réseau coronarien soulève un certain nombre de difficultés.

Tout d'abord, l'amplitude du signal auriculaire est beaucoup plus faible que celle du signal ventriculaire, et il est nécessaire, lors de la détection de ce signal, de réduire au minimum les parasites, bruits ou autres brouillages qui pourraient se superposer au signal utile.

Par ailleurs, du point de vue mécanique, le diamètre extérieur de la sonde doit être le plus uniforme possible, de manière à limiter les difficultés d'extraction si la sonde doit être retirée - l'extraction d'une sonde hors du réseau coronarien est en effet beaucoup plus délicate que celle d'une sonde endocavitaire introduite par le réseau veineux périphérique.

D'autre part, l'introduction d'une sonde dans le réseau coronarien, opérée par des voies endocavitaires, est une intervention particulièrement délicate, compte tenu notamment du fait que la position des points de stimulation est très importante. Dans le cas d'un stimulateur de type "multisite", les points de stimulation des cavités droites et des cavités gauches doivent être les plus éloignés possible pour optimiser la resynchronisation de l'ensemble des cavités cardiaques.

Ce type particulier de sonde doit pour cette raison répondre à un certain de critères précis :
- présence d'un canal interne axial pour l'enfilage d'un mandrin dans la sonde au moment de l'implantation, notamment pour la recherche de l'entrée du sinus coronaire,
- minimisation des éléments rigides qui pourraient gêner la progression de la sonde dans le réseau coronarien après que le chirurgien ait trouvé l'entrée du sinus coronaire et introduit l'extrémité de la sonde dans celui-ci,
- de façon générale, une nouvelle sonde doit être suffisamment proche dans son maniement des sondes existantes (mise en place par mandrin, préformé ou non, sous amplificateur de brillance, etc.) afin qu'elle soit acceptée sans difficulté par les chirurgiens qui auront à l'implanter.

D'autre part, du point de vue industriel, il est souhaitable de rationaliser la fabrication d'une telle sonde afin d'en réduire le coût final, ce qui implique de:
- limiter la complexité de l'électrode de stimulation en carbone, car il s'agit d'un matériau difficile à usiner,
- concevoir la sonde de manière qu'elle puisse être réalisée à partir de matériaux classiques et assemblée par des techniques connues et éprouvées,
- assurer la fiabilité de l'assemblage, tant en ce qui concerne l'étanchéité de la sonde que sa tenue mécanique à long terme.

Essentiellement, la présente invention propose de réaliser la sonde coronaire auriculaire gauche en utilisant la technique des sondes tripolaires (une électrode de stimulation annulaire entourée de deux électrodes de référence, également annulaires, reliées au même potentiel) afin de pallier la difficulté mentionnée en premier lieu, à savoir le risque de détection de signaux parasites importants compte tenu de l'amplitude du signal auriculaire beaucoup plus faible que celle du signal ventriculaire.

L'intérêt des sondes tripolaires est en particulier exposé dans l'article de Callaghan F. et coll., *Spatial Discrimination of Cardiac Signals*, REM, Vol. 6, n° 3, p.223, qui montre l'intérêt de la détection tripolaire appliquée à la détection d'un électrogramme endocavitaire auriculaire, cette technique procurant un rapport signal/bruit très supérieur à ce qu'il est possible d'obtenir avec une détection unipolaire ou bipolaire.

Cette configuration est également décrite dans le EP-A-0 159 753, appliquée à des électrodes endocavitaires ou épicardiques, et qui comprend les caractéristiques énoncées dans le préambule de la revendication 1.

La présente invention, quant à elle, propose de réaliser une sonde de stimulation de l'oreillette gauche implantable dans le réseau veineux coronarien, et comprenant les éléments énoncés dans la partie caractéristique de la revendication 1. Des mises en oeuvre avantageuses sont présentées dans les sous-revendications.

On va maintenant décrire un exemple de mise en oeuvre de l'invention, en référence aux dessins annexés.

La figure 1 est une vue d'ensemble de l'extrémité de la sonde selon l'invention.

La figure 2 est une vue partiellement coupée de la sonde de la figure 1 dans la région des électrodes.

La figure 3 est une vue éclatée montant les différents éléments représentés assemblés sur la figure 2.

La figure 4 est une vue montrant, isolément, le joint de silicone et sa position par rapport à l'électrode de stimulation.

La figure 5 est une vue éclatée coupée reprenant les différents éléments présentés sur la vue de la figure 3.

Sur les figures, où les mêmes références numériques désignent des éléments identiques, la référence 10 désigne de façon générale la partie distale de la sonde, destinée à être introduite par voie endocavitaire dans le réseau coronaire veineux. L'extrémité distale 12 de cette partie porte éventuellement (mais tel n'est pas l'objet de l'invention) une électrode de stimulation du ventricule gauche, non représentée. Entre cette extrémité distale 12 et une région plus proximale 14 se situe une région 16 portant un ensemble d'électrodes destinées à la stimulation (et au recueil) de l'oreillette gauche.

Cette configuration d'électrodes est une configuration, en elle-même connue, de type tripolaire, avec une électrode dite de stimulation 18 (cette électrode servant également, bien évidemment, au recueil des signaux auriculaires) encadrée par deux électrodes de référence 20, 22 équipotentielles.

La sonde comporte, de manière en elle-même également classique, deux conducteurs 24, 26, qui sont des conducteurs coaxiaux spiralés, afin de laisser le maximum de souplesse à la sonde.

Le schéma de connexion est avantageusement celui correspondant à la norme française et européenne NF EN 50077 *"Connecteur à bas profil pour stimulateurs cardiaques implantables"*, qui définit un système de connexion normalisé dit "IS-1" permettant de garantir l'interchangeabilité des sondes et des générateurs d'impulsions produits par différents fabricants (l'invention n'est toutefois pas limitée au cas particulier des systèmes selon cette norme).

L'électrode de stimulation 18 est réalisée en carbone, et elle présente une surface de l'ordre de 2 à 4 mm². Chacune des électrodes de référence 20, 22 est constituée d'une bague en platine iridié d'une surface d'environ 10 mm² et située à environ 10 mm de part et d'autre de l'électrode de stimulation 18. Les conducteurs 24 et 26 sont, de manière classique, réalisés en acier inoxydable ou un autre alliage, notamment un MP35N, et ils sont isolés entre eux par une gaine 28 constituée d'un tube de silicone, le tout étant isolé de l'extérieur par une gaine externe 30, également en silicone, d'un diamètre de 2,5 mm environ.

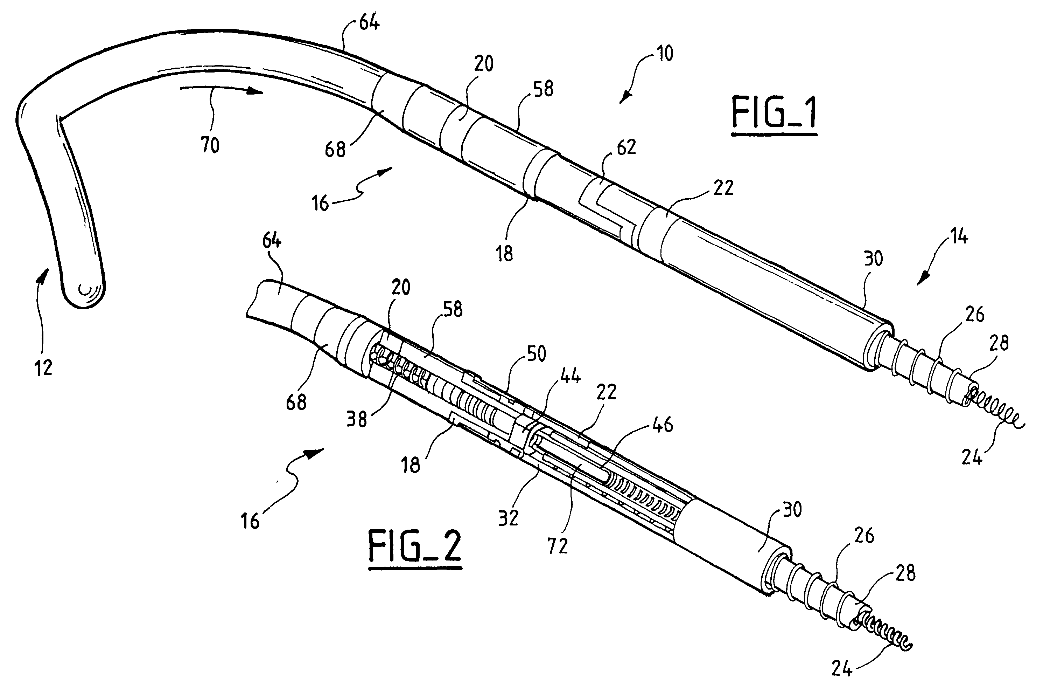

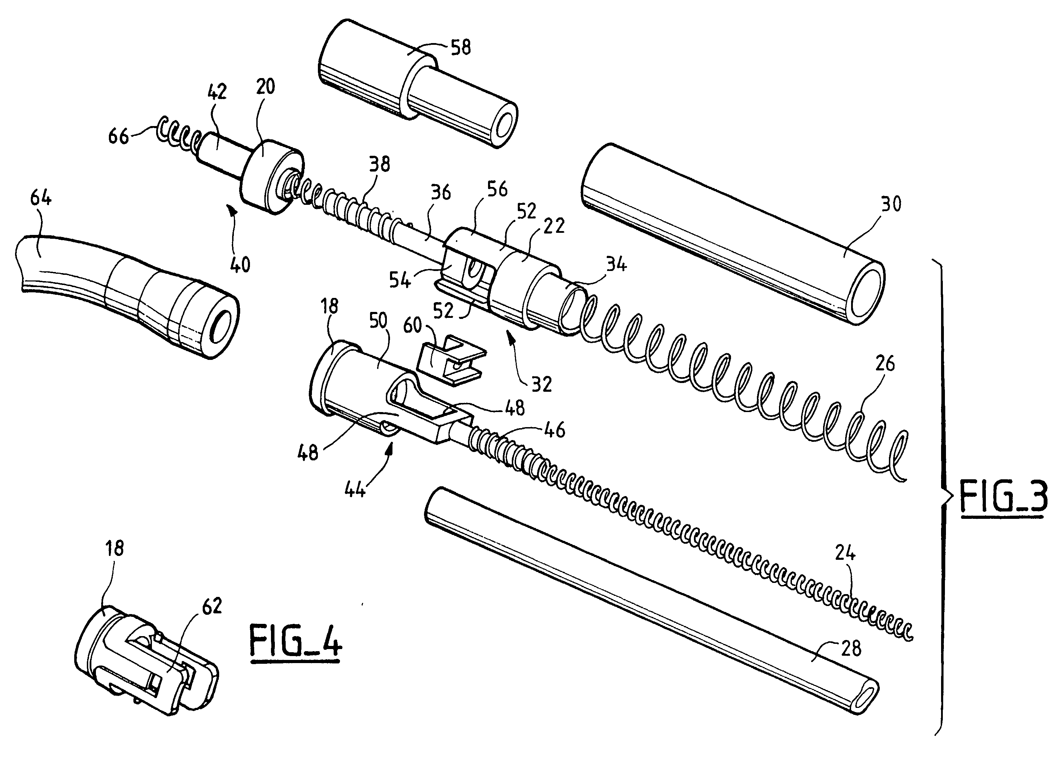

Les figures 2, 3 et 5 montrent la structure interne de la sonde dans la région 16 des électrodes, notamment la manière dont est réalisée la liaison électrique entre les électrodes 18, 20 et 22 et les conducteurs 24 et 26.

La structure que l'on va décrire, qui n'est cependant donnée qu'à titre d'exemple non limitatif, permet, comme on le comprendra, d'offrir un certain nombre d'avantages tout à fait déterminants dans le cas d'une sonde destinée à être introduite dans le réseau coronaire, notamment :
- sonde monodiamètre, c'est-à-dire qui ne présente pas de surépaisseur dans la région des électrodes : en effet, en cas de développement de fibroses, il est important que le diamètre de la sonde soit le plus uniforme, le plus fin et le plus lisse possible ;
- présence d'un canal interne axial autorisant l'enfilage d'un mandrin pour l'implantation : ce canal interne, dont le diamètre est typiquement de l'ordre de 0,6 mm, s'étend sur toute la partie proximale de la sonde et se prolonge sur toute la région des électrodes et au-delà jusqu'à l'extrémité distale 12 ;
- une fois le mandrin retiré, la sonde doit rester la plus souple possible pour ne pas gêner sa progression dans le sinus coronaire, notamment en réduisant au minimum la longueur des éléments rigides ;
- tenue mécanique à long terme, étanchéité et tenue au claquage, en particulier en cas d'application de chocs de défibrillation ;
- simplicité de montage, en évitant d'avoir à mettre au point de nouvelles technologies d'assemblage : on verra que le montage des diverses pièces de la sonde se fait par des techniques classiques de collage silicone, sertissage, coincement, soudure, etc.

Les figures 2, 3 et 5 montrent les différentes pièces constituant la sonde de l'invention dans la région 16 des électrodes.

Il s'agit essentiellement de réaliser une bifurcation mécanique et électrique permettant d'opérer la liaison électrique entre les deux électrodes de référence 20 et 22 et au conducteur externe 26, d'une part, et celle de l'électrode de stimulation 18 au conducteur interne 24.

À cet effet, on prévoit une pièce métallique 32 dont une surface externe annulaire constitue l'électrode de référence proximale 22. Cette pièce est prolongée en direction proximale par une partie tubulaire 34 permettant de réaliser la liaison électrique au conducteur externe spiralé 26, par une technique classique de coincement après élargissement local de ce conducteur.

En ce qui concerne l'électrode de référence distale 20, elle est reliée à un tube métallique creux 36 sur lequel est vissé un conducteur spiralé 38 de diamètre sensiblement inférieur au conducteur externe 26 ; ce conducteur 38 est lui-même relié dans sa région distale à une pièce métallique 40 par vissage/sertissage dans une partie tubulaire creuse 42, une surface externe de la pièce 40 constituant l'électrode de référence distale 20.

On dispose ainsi de deux sous-ensembles, l'un 26, 32 portant l'électrode de référence proximale 22 et l'autre 36, 38, 40 portant l'électrode de référence distale 20. Ces deux sous-ensembles seront réunis dans une étape ultérieure que l'on décrira plus bas.

L'électrode de stimulation 18 est portée par une pièce creuse métallique 44, l'électrode 18 en carbone étant par exemple sertie à l'intérieur de cette pièce 44 selon une technique en elle-même connue et déjà appliquée par exemple aux sondes à vis rétractable telles que la sonde UR45 d'ELA Médical.

La pièce 44 est prolongée en direction proximale par un tube cylindrique creux 46 sur lequel est monté par vissage/sertissage le conducteur interne 24. Le tube 46 se prolonge en direction distale par deux branches 48, 48 diamétralement opposées se rejoignant sur un cylindre 50 sensiblement plus grand et dans lequel est sertie l'électrode annulaire, ou partielment annulaire (c'est-à-dire sectorielle), de stimulation 18.

Le sous-ensemble constitué par la pièce 44 et le conducteur interne 24 revêtu de sa gaine isolante 28 est monté dans le sous-ensemble, précédemment décrit, constitué de la pièce 32 et du conducteur externe 26.

Le sous-ensemble constitué par la pièce 40, du conducteur 38 et du cylindre 36, est alors monté sur ces deux sous-ensembles de la manière suivante.

La pièce 32 est prolongée en direction distale par deux branches 52, 52 diamétralement opposées et décalées angulairement d'environ 90° par rapport aux branches 48, 48 de la pièce 44, de manière à permettre une interpénétration sans contact de ces pièces 32 et 44. Un noyau métallique 54, usiné avec le tube 36, est alors placé entre les deux branches 52, 52 de la pièce 32, et lié mécaniquement et électriquement à celle-ci par exemple par soudure laser en 56.

On prévoit par ailleurs d'insérer une entretoise 60 en matériau isolant pour maintenir et caler les différents éléments des sous-ensembles ainsi assemblés avant injection de colle ou surmoulage.

Avant cet assemblage, on aura enfilé sur le cylindre 36 un manchon d'isolement 58 en matériau silicone assurant le positionnement en direction axiale et l'isolement électrique entre l'électrode de référence distale 20 et l'électrode de stimulation 18 ; ce manchon 58 présente une partie épaulée de plus petit diamètre pénétrant à l'intérieur du tube 50 jusqu'à venir en butée contre le noyau 54.

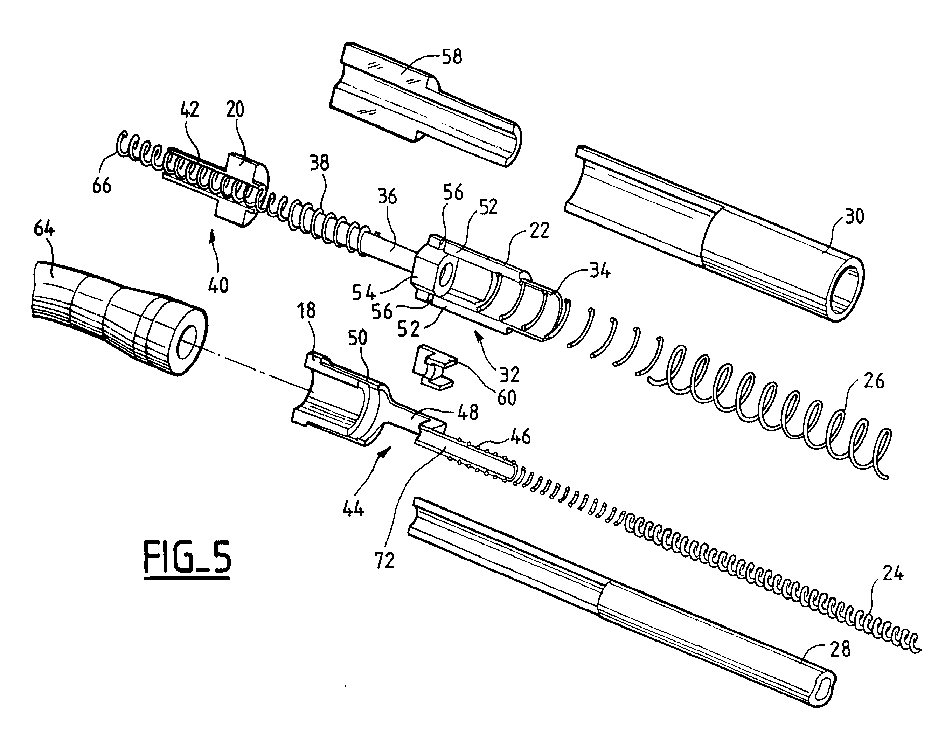

La surface externe de la pièce 44 et les deux branches 52, 52 de la pièce 32 sont isolées électriquement entre elles par un joint 62 (figure 4) en matériau silicone, moulé en place selon une technique en elle-même connue, déjà utilisée pour la fabrication des sondes à vis rétractable. Ce joint de colle 62 résultant assure alors l'isolement, l'étanchéité et la tenue mécanique des diverses pièces dans la région des électrodes.

L'ensemble est ensuite pourvu d'un tube silicone 64 constituant l'extrémité distale de la sonde, qui est avantageusement un tube à double courbure renforcé par la partie 66 du conducteur 38 prolongé en direction distale. Enfin, on peut placer un collier 68 chargé d'un agent stéroïde, en position distale par rapport aux trois électrodes 18, 20 et 22. Dans la mesure où la sonde est implantée dans le réseau coronarien veineux, le flux sanguin est orienté, comme illustré par la flèche 70, dans le sens proximal par rapport à la sonde, de sorte que ce flux se chargera de drainer le principe actif diffusé par le collier 68 vers les tissus cibles situés en aval.

## Revendications

1. Une sonde (10) de stimulation d'une cavité cardiaque par un dispositif médical implantable actif, notamment un stimulateur cardiaque, défibrillateur et/ou cardioverteur implantable, cette sonde étant une sonde de type tripolaire comportant un corps cylindrique pourvu, dans une région (16) située axialement à distance de son extrémité distale (12), de :
- une électrode de stimulation annulaire ou sectorielle (18) reliée électriquement à un conducteur interne d'un conducteur coaxial (24) de la sonde, et
- deux électrodes de référence annulaires (20, 22), distale (20) et proximale (22), reliées électriquement entre elles et à un conducteur externe d'un conducteur coaxial (26) de la sonde, l'électrode de stimulation étant disposée axialement entre les deux électrodes de référence,
un premier organe de liaison électrique reliant les deux électrodes de référence (20, 22) à un segment conducteur interne (36, 38) disposé dans la région de l'électrode de stimulation mais électriquement isolé de cette dernière.
***caractérisée en ce que**,* cette sonde étant apte à être implantée dans le réseau veineux coronarien pour permettre la stimulation de l'oreillette gauche, ledit premier organe de liaison électrique (32) comporte :
- une liaison électrique entre une région (34) radialement en périphérie, reliée au conducteur externe (26), et une région (54) radialement plus proche de l'axe de la sonde, reliée audit segment conducteur interne (36, 38), et
- une première pièce avec un corps tubulaire portant l'une (22) des deux électrodes de référence, ce corps tubulaire étant prolongé en direction axiale par deux branches (52) diamétralement opposées portant entre elles un noyau (54) central auquel elles sont mécaniquement et électriquement reliées, ce noyau étant lui-même relié à l'autre (20) des deux électrodes de référence par ledit segment conducteur interne (36, 38) disposé dans la région de l'électrode de stimulation.

2. La sonde de la revendication 1, comportant un second organe de liaison électrique (44), comportant une liaison électrique entre une région (50) radialement en périphérie, reliée à l'électrode de stimulation (18), et une région (46) radialement plus proche de l'axe de la sonde, reliée au conducteur de petit diamètre (24).

3. La sonde de la revendication 2, dans laquelle ledit second organe de liaison électrique (44) comporte une seconde pièce avec un corps tubulaire portant l'électrode de stimulation (18), ce corps tubulaire étant prolongé en direction axiale par deux branches (48) diamétralement opposées, mécaniquement et électriquement reliées entre elles et au conducteur interne (24) de la sonde.

4. La sonde de la revendication 3, dans laquelle les branches respectives (48 ; 52) des deux pièces avec un corps tubulaire s'étendent axialement dans des sens opposés et sont décalées angulairement d'environ 90° de manière à permettre une interpénétration sans contact des deux pièces.

5. La sonde de la revendication 4, dans laquelle les zones en vis-à-vis des branches dans la région d'interpénétration sont maintenues à distance et électriquement isolées par un joint (62) moulé en place.

6. La sonde de la revendication 4, comprenant une entretoise (60) en matériau électriquement isolant apte à assurer le maintien à distance des zones en vis-à-vis des branches dans la région d'interpénétration.

7. La sonde de la revendication 1, dans laquelle le diamètre extérieur de la sonde est essentiellement constant, sans surépaisseur dans la région des électrodes.

8. La sonde de la revendication 1, comportant un canal interne axial (72) s'étendant de façon continue depuis l'extrémité proximale jusqu'à un point situé au-delà de ladite région des électrodes.

9. La sonde de la revendication 1, dans laquelle le segment conducteur (36, 38) est un segment déformable au moins sur une partie de sa longueur.

10. La sonde de la revendication 1, comportant en outre un collier (68) porteur d'un agent stéroïde, monté dans une région située à proximité, en; direction distale, de la région des électrodes.

## Patentansprüche

1. Sonde (10) zur Stimulation einer Herzkammer durch eine aktive implantierbare medizinische Vorrichtung, insbesondere ein implantierbarer Herzschrittmacher, Defibrillator und/oder Kardioverter, wobei diese Sonde eine Sonde vom dreipoligen Typ ist, der einen zylindrischen Körper aufweist, der in einem Bereich (16), welcher axial von ihrem distalen Ende (12) entfernt liegt, versehen ist mit:
- einer ringförmigen oder sektorförmigen Stimulationselektrode (18), die mit einem internen Leiter eines koaxialen Leiters (24) der Sonde elektrisch verbunden ist, und
- zwei ringförmigen Referenzelektroden (20, 22), einer distalen (20) und einer proximalen (22), die miteinander und mit einem externen Leiter eines koaxialen Leiters (26) elektrisch verbunden sind, wobei die Stimulationselektrode axial zwischen den beiden Referenzelektroden angeordnet ist,
wobei ein erstes elektrisches Verbindungsorgan die zwei Referenzelektroden (20, 22) mit einem internen Leitersegment (36, 38) verbindet, welches im Bereich der Stimulationselektrode angeordnet ist, aber gegenüber dieser elektrisch isoliert ist,
**dadurch gekennzeichnet, dass** diese Sonde dafür geeignet ist, in das koronare Venennetz implantiert zu werden, um die Stimulation des linken Herzvorhofs zu ermöglichen, wobei das erste elektrische Verbindungsorgan (32) aufweist:
- eine elektrische Verbindung zwischen einem Bereich (34), der radial gesehen in der Peripherie liegt und der mit einem externen Leiter (26) verbunden ist, und einem Bereich (54), der radial gesehen näher an der Achse der Sonde liegt und der mit dem internen Leitersegment (36, 38) verbunden ist, und
- ein erstes Teil mit einem röhrenförmigen Körper, das eine (22) der zwei Referenzelektroden trägt, wobei dieser röhrenförmige Körper in axialer Richtung durch zwei sich diametral gegenüberliegende Arme (52) verlängert ist, die zwischen sich einen Mittelkern (54) tragen, mit dem sie mechanisch und elektrisch verbunden sind, wobei dieser Kern selber mit der anderen (20) der beiden Referenzelektroden durch das interne Leitersegment (36, 38) verbunden ist, das im Bereich der Stimulationsclcktrode angeordnet ist.

2. Sonde gemäß Anspruch 1, aufweisend ein zweites elektrisches Verbindungsorgan (44), das eine elektrische Verbindung zwischen einem Bereich (50), der radial gesehen in der Peripherie liegt und der mit der Stimulationselektrode (18) verbunden ist, und einem Bereich (46), der radial gesehen näher an der Achse der Sonde liegt und der mit dem Leiter von kleinem Durchmesser (24) verbunden ist.

3. Sonde gemäß Anspruch 2, in der das zweite elektrische Verbindungsorgan (44) ein zweites Teil mit einem röhrenförmigen Körper aufweist, das die Stimulationselektrode (18) trägt, wobei dieser röhrenförmige Körper in axialer Richtung durch zwei sich diametral gegenüberliegende Arme (48) verlängert ist, die mechanisch und elektrisch miteinander und dem internen Leiter (24) der Sonde verbunden sind.

4. Sonde gemäß Anspruch 3, in der sich die jeweiligen Arme (48; 52) der zwei Teile mit röhrenförmigem Körper axial in entgegen gesetzte Richtungen erstrecken und die ungefähr mit einem Winkel von 90° versetzt sind, um eine gegenseitige Penetration ohne Kontakt der beiden Teile zu erlauben.

5. Sonde gemäß Anspruch 4, in der die Zonen gegenüber den Armen im Bereich der gegenseitigen Penetration auf Distanz gehalten werden und elektrisch durch eine dort anliegende Fuge (62) isoliert sind.

6. Sonde gemäß Anspruch 4, die einen Abstandshalter (60) aus elektrisch isolierendem Material aufweist, der dazu geeignet ist, sicher zu stellen, dass die Zonen gegenüber den Armen im Bereich der gegenseitigen Penetration auf Distanz gehalten werden.

7. Sonde gemäß Anspruch 1, in der der äußere Durchmesser der Sonde im wesentlichen konstant ist, ohne Überdicke im Bereich der Elektroden.

8. Sonde gemäß Anspruch 1, die einen internen axialen Kanal (72) aufweist, der sich auf durchgehende Weise vom proximalen Ende bis zu einem Punkt erstreckt, der sich jenseits des Elektrodenbereichs befindet.

9. Sonde gemäß Anspruch 1, in der das Leitersegment (36, 38) ein verformbares Segment ist, zumindest auf einem Teil seiner Länge.

10. Sonde gemäß Anspruch 1, die darüber hinaus eine Lasche (68) aufweist, die ein steroides Mittel trägt und die in einem Bereich befestigt ist, der sich in distaler Richtung in der Nähe des Bereichs der Elektroden befindet.

## Claims

1. A lead for stimulating a cardiac cavity by an active implantable medical device, in particular a cardiac implantable pacemaker, defibrillator and/or cardioverter, said lead being a lead of the tripolar type including a cylindrical body provided, in an area (16) located axially remote from its distal end (12), with:
- an annular or sectorial stimulation electrode (18) electrically connected to an internal conductor of a coaxial conductor (24) of the lead, and
- two annular reference electrodes (20, 22), distal (20) and proximal (22), electrically connected together and to an external conductor of a coaxial conductor (26) of the lead, the stimulation electrode being axially arranged between the two reference electrodes,
a first electrical connection member connecting the two reference electrodes (20, 22) to an internal conductor segment (36, 38) that is arranged in the area of the stimulation electrode but electrically insulated from the latter,
***characterised in that**,* said lead being adapted to be implanted in the coronary venous network for stimulation of the left atrium, said first electrical connection member (32) includes :
- an electrical connection between a radially peripheral area (34), connected to the external conductor (26) and an area (54) radially closer to the axis of the lead, connected to said internal conductor segment (36, 38), and
- a first part with a tubular body carrying one (22) of the two reference electrode, said tubular body being axially extended by two diametrically opposed branches (52) carrying therebetween a central core (54) to which they are mechanically and electrically connected, said core being itself connected to the other (20) of the two reference electrodes by the internal connector segment (36, 38) arranged in the area of the stimulation electrode.

2. The lead of claim 1, comprising a second electrical connection body (44), comprising an electrical connection between a radially peripheral area (50), connected to the stimulation electrode (18), and an area radially closer to the axis of the lead (i.e., smaller in dimension than the first radial area on the same axis), connected to the small diameter conductor (24).

3. The lead of claim 2, wherein said second electrical connection body (44) comprises a second part with a tubular body carrying the stimulation electrode (18), said tubular body being axially extended by two diametrically opposed branches (48) mechanically and electrically connected therebetween and to the internal conductor (24) of the lead.

4. The lead of claim 3, wherein the respective branches (48, 52) of the two parts with a tubular body axially extend in opposite directions and are angularly shifted by approximately 90° so as to allow an interpenetration of the two parts without contact.

5. The lead of claim 4, wherein the opposing areas of the branches in the area of interpenetration are maintained remotely from one another and electrically isolated by a seal (62) moulded *in situ*.

6. The lead of claim 4, comprising a spacer (60) in electrically insulating material, adapted to remotely maintain the opposing areas of the branches in the area of interpenetration.

7. The lead of claim 1, wherein the external diameter of the lead is essentially constant, without any extra thickness in the area of the electrodes.

8. The lead of claim 1, comprising an axial internal lumen (72) continuously extending from the proximal end up to a point located beyond the area of the electrodes.

9. The lead of claim 1, wherein the internal conductor segment (36, 38) is a segment deformable over at least a part of its length.

10. The lead of claim 1, further comprising a collar (68) carrying a steroid agent, mounted in an area located in the vicinity, in the distal direction, of the electrode area.
